# EUROPEAN PATENT SPECIFICATION

(11) **EP 2 006 680 B1**
(45) Date of publication and mention of the grant of the patent: **16.04.2014**
(21) Application number: 07714417.8
(22) Date of filing: 16.02.2007
(51) Int. Cl.: G01N 33/68

(54) **BIOLOGICAL LOAD INDICATOR AND METHOD OF MEASURING BIOLOGICAL LOAD**
INDIKATOR UND MESSVERFAHREN FÜR BIOLOGISCHE LAST
INDICATEUR D'UNE CHARGE BIOLOGIQUE ET PROCEDE PERMETTANT DE MESURER LA CHARGE BIOLOGIQUE

(30) Priority: 17.02.2006 JP 2006041633
(43) Date of publication of application: 24.12.2008
(60) Divisional application: 12154982.8
(73) Proprietor: Sekiyama, Atsuo, Suita-shi, Osaka 5640063 (JP)
(72) Inventor: Sekiyama, Atsuo, Suita-shi, Osaka 5640063 (JP)
(74) Representative: Duckworth, Timothy John
(86) International application number: PCT/JP2007/052887
(87) International publication number: WO 2007/094472

(56) References cited:
- WO-A1-2006/003927
- JP-A- 2001 194 369
- JP-A- 2005 124 565
- US-A1- 2005 245 557
- OSTROWSKI KENNETH ET AL: "Pro- and anti-inflammatory cytokine balance in strenuous exercise in humans" JOURNAL OF PHYSIOLOGY (CAMBRIDGE), vol. 515, no. 1, 15 February 1999 (1999-02-15), pages 287-291, XP002538341 ISSN: 0022-3751
- KAESTNER F ET AL: "Different activation patterns of proinflammatory cytokines in melancholic and non-melancholic major depression are associated with HPA axis activity" JOURNAL OF AFFECTIVE DISORDERS, ELSEVIER BIOCHEMICAL PRESS, AMSTERDAM, NL, vol. 87, no. 2-3, 1 August 2005 (2005-08-01), pages 305-311, XP004992613 ISSN: 0165-0327
- SCHWEITZER B ET AL: "Multiplexed protein profiling on microarrays by rolling-circle amplification" NATURE BIOTECHNOLOGY, NATURE PUBLISHING GROUP, NEW YORK, NY, US, vol. 20, no. 4, 1 April 2002 (2002-04-01), pages 359-365, XP002362307 ISSN: 1087-0156

## Description

### Technical Field

The present invention relates to biological load indicators and methods for measuring biological loads. Specifically, the present invention relates to the technical field for objectively evaluating biological loads such as stress and fatigue by molecular-biological approaches.

### Background Art

Mental stress and/or physical stress affects host defense mechanisms including nervous, endocrine and immune systems (see non-patent references 1 and 2). Various cytokines (such as IL-1β, IL-6, and TNF-α) are upregulated by stress (see non-patent references 1 and 3). This suggests that cytokines are likely to be involved in interference with host defense (see non-patent reference 4). However, the molecular mechanism of the induction of host defense-reducing cytokines by stress is not fully understood.

Interleukin-18 (IL-18) is a cytokine that was discovered as an interferon-γ (IFN-γ)-inducing factor (see non-patent reference 5, patent reference 1). IL-18 has various biological activities such as Fas ligand induction, elevation of the cytolytic activity of T cells (see non-patent reference 6), and production of IL-4 and IL-13 (see non-patent reference 7). IL-18 activates Toll-like receptor 2 (see non-patent reference 9) and myeloid differentiation protein (Myd)-88 (see non-patent reference 10). These activations are necessary for IL-6 induction (see non-patent reference 11). Therefore, IL-18 is involved in the production of both Th1 and Th2 cytokines (see non-patent reference 12).

IL-18 is produced as a 24 kD precursor protein that is processed to a 18 kD mature active form by an IL-1β converting enzyme (ICE, also called caspase-1) (see non-patent reference 13). Caspase-1 is induced as an inactive precursor protein procaspase-1 that is activated by caspase-11 (see non-patent reference 14). It is reported that caspase-11 mRNA expression involves trans-activation of NF-κB (see non-patent reference 15), which is mediated by P38MAP kinase (see non-patent reference 16). It is also reported that the induction of caspase-11 mRNA by LPS (lipopolysaccharide) and the following activation of caspase-1 are inhibited by SB203580, a P38MAP kinase inhibitor, in a glioma cell line C6 (see non-patent reference 17).

Recent studies report that IL-18 mRNA is expressed in an adrenal gland in response to an adrenocorticotropic hormone (ACTH) and cold stress (see non-patent reference 18). It is also reported that different promoters are used for IL-18 mRNA expression in an adrenal gland and an immune cell (see non-patent reference 19). However, both studies described above are silent on the induction of mature IL-18. On the other hand, it is reported that IL-18 in blood plasma increases in psychiatric patients (see non-patent reference 20).

In society today, people work and live under various types of stress. In general, there are differences in sensory perception of stress among individuals, and there is no specific indicator of the presence or absence of stress or the intensity of stress. As a result of investigations, the inventor has found that stress causes an increase in cytokines such as IL-18 and have revealed the flow of signal transduction with IL-18 at the top of the stress cascade, so that stress can be objectively evaluated (see patent reference 2, non-patent references 21 - 23).
patent reference 1: JP-A-8-193098
patent reference 2: WO2006/003927
non-patent reference 1: Dugue, B. et al. Scand. J. Clin. Invest. 53, 555-561 (1993)
non-patent reference 2: Kiecolt-Glaser, J. K. et al. Proc. Natl. Acad. Sci. USA 93, 3043-3047 (1996)
non-patent reference 3: Endocrinology 133, 2523-2530 (1993)
non-patent reference 4: Schubert, C. et al. Psychosom. Med. 61, 876-882 (1999)
non-patent reference 5: Zhou, D. et al. Nature 378, 88-91 (1995)
non-patent reference 6: Nakanishi, K. et al. Annu. Rev. Immunol. 19 423-474 (2001)
non-patent reference 7: Hoshino, T. et al. J. Immunol. 162, 5070-5077 (1999)
non-patent reference 8: Dinarello, C.A. et al. J. Leukoc. Biol. 63, 658-664 (1998)
non-patent reference 9: Blease, K. et al. Inflamm. Res. 50, 552-560 (2001)
non-patent reference 10: Adachi, O. et al. Immunity 9, 143-150 (1998)
non-patent reference 11: Takeuchi, O. et al. J. Immunol. 165, 5392-5396 (2000)
non-patent reference 12: Hoshino, T. et al. J. Immunol. 166, 7014-7018 (2001)
non-patent reference 13: Gu, Y. et al. Science 275, 206-209 (1997)
non-patent reference 14: Wang, S. et al. Cell 92, 501-509 (1998)
non-patent reference 15: Schauvliege, R. et al. J. Biol. Chem. 277, 41624-41630 (2002)
non-patent reference 16: Vanden Berghe, W. et al. J. Biol. Chem. 273, 3285-3290 (1998)
non-patent reference 17: Hur, J. et al. FEBS Lett. 507, 157-162 (2001)
non-patent reference 18: Conti, B. et al. J. Biol. Chem. 272, 2035-2037 (1997)
non-patent reference 19: Sugama, S. et al. J. Immunol. 165, 6287-6292 (2000)
non-patent reference 20: Kokai, M. et al. J. Immunother. 25, 68-71 (2002)
non-patent reference 21: Sekiyama, A. et al. Immunity 22(6), 669-677 (2005)
non-patent reference 22: Sekiyama, A. et al. J Neuroimmunol. 171(1-2), 38-44 (2006)
non-patent reference 23: Sekiyama, A. et al. J Med Invest. 52, 236-239 (2005)

### Disclosure of the Invention

### Problems to be Solved by the Invention

A living organism has a mechanism to keep its biological functions constant in response to loads and stimuli. Such a mechanism is called homeostasis or host-defense mechanism. It is known that mental, physiological, physical, or chemical stress and/or fatigue acts as a load on homeostasis or host-defense mechanism and that various diseases are caused by breakdown of homeostasis or host-defense mechanism.

A load on homeostasis or host-defense mechanism, such as stress, fatigue and blues can further cause mental disorders, physical disorders, and even suicide, and is a serious challenge to health. However, evaluation of such conditions basically accompanied by subjective symptoms has been possible only by self-assessment. It has also been very difficult to detect abnormalities by known biochemical or psychological tests, and therefore, it has been impossible to make objective evaluations, grasp severity or develop some remedies. Various biochemical or physiological indicators have been proposed. However, hormones or amines are instable and difficult to be quantified, and therefore, they are not suitable as indicators, although their level can change in conjunction with stress.

An object of the present invention is to provide techniques capable of objectively and specifically evaluating various loads on living organisms, of which evaluation was conventionally possible only by subjective symptom-dependent methods, such as fatigue. Since living organisms feel discomfort when they recognize loads on biological homeostasis and host-defense mechanism, the object of the present invention necessarily encompasses to provide techniques capable of objectively and specifically evaluating a feeling of discomfort or comfort in living organisms.

### Means for Solving the Problems

In light of the problems described above, the inventor has made active investigations and has found that an exhaustive survey of variations in the expression of cytokines or chemokines constituting an IL-18-centered biological cascade allows objective understanding of various biological loads such as stress and fatigue, so that the invention has been completed.

Accordingly, the present invention provides the following.
[1] Use in vitro of an indicating agent in evaluating mental conditions selected from the group consisting of mental fatigue, physical fatigue, stress, anxiety, depression and schizophrenia, which indicating agent comprises at least eight kinds of cytokines selected from the group consisting of IL-1β, IL-1ra, IL-2, IL-3, IL-4, IL-5, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, wherein the amount of each cytokine is multiplied by a weighting coefficient.
[2] Use in vitro of a test agent in testing mental conditions, which mental conditions are selected from the group consisting of mental fatigue, physical fatigue, stress, anxiety, depression and schizophrenia, which test agent comprises at least eight kinds of antibodies selected from the group consisting of antibodies specifically recognizing IL-1β, IL-1ra, IL-2, IL-3, IL-4, IL-5, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-α, neurotrophin 5, MCP-3, β-2-microglobulin, angiotensin II, CSF-3, CXC chemokine ligand 1, CXC chemokine ligand 5 and HGF, respectively.
[3] A method of measuring mental conditions selected from the group consisting of mental fatigue, physical fatigue, stress, anxiety, depression and schizophrenia, which comprises a step of measuring the level of the cytokines in a biological sample using the test agent of the above-mentioned [2], and a step of comparing the amount obtained in the aforementioned measurement step with that of the indicating agent of claim 1 by proportional weighting.
[4] The method of the above-mentioned [3], wherein the aforementioned biological sample is plasma, serum, saliva or urine.

### Effects of the Invention

Use in vitro of the stress or fatigue indicating agent comprising cytokines in accordance with the present invention allows objective and molecular-biological evaluation of the degree of stress or fatigue in a mammal such as human. Use in vitro of the stress or fatigue test agent comprising antibodies capable of recognizing the cytokines allows an objective and quantitative test of the degree of stress or fatigue in a mammal such as human. The stress or fatigue measurement method of the invention including measuring the concentration of cytokines in a biological sample with the test agent allows objective and quantitative measurement of the degree of stress or fatigue in a mammal such as human.

Therefore, the present invention is useful for reconstruction of the basis of preventive medicine and public health administration, evaluation and control of life activity level and mental health of patients under medial treatment, evaluation and control of life activity level and mental health of convalescent patients, determination of the range of industrial or occupational health or work-related injuries and illness, evaluation of working environments, evaluation of working intensity, assessment of living or working environments, evaluation of school sanitation or learning environments or intensity, environmental assessment, evaluation of diseases or disorders mainly characterized by stress or fatigue, international unification of stress evaluation criteria, and establishment of international criteria for evaluation of each of the above.

### Brief Description of the Drawings

[Fig. 1a] Fig. 1a is a graph showing a correlation pattern of levels of blood plasma cytokines or chemokines in a total of 402 healthy subjects (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation, and the same applies to the other correlation tables described below).
[Fig. 1b] Fig. 1b is a graph showing a correlation pattern of levels of pre-night shift blood plasma cytokines or chemokines in 142 subjects who are healthy but frequently work night shifts.
[Fig. 1c] Fig. 1c is a graph showing a correlation pattern of post-night shift blood plasma cytokines or chemokines in the subjects.
[Fig. 1d] Fig. 1d is a classification table obtained by a GO TO PAGE 13 logistic regression analysis to determine whether it is possible to correctly classify 90 people randomly sampled from the subjects into a pre-night shift group and a post-night shift group according to the present invention, in which the accuracy is 88% according to the invention.

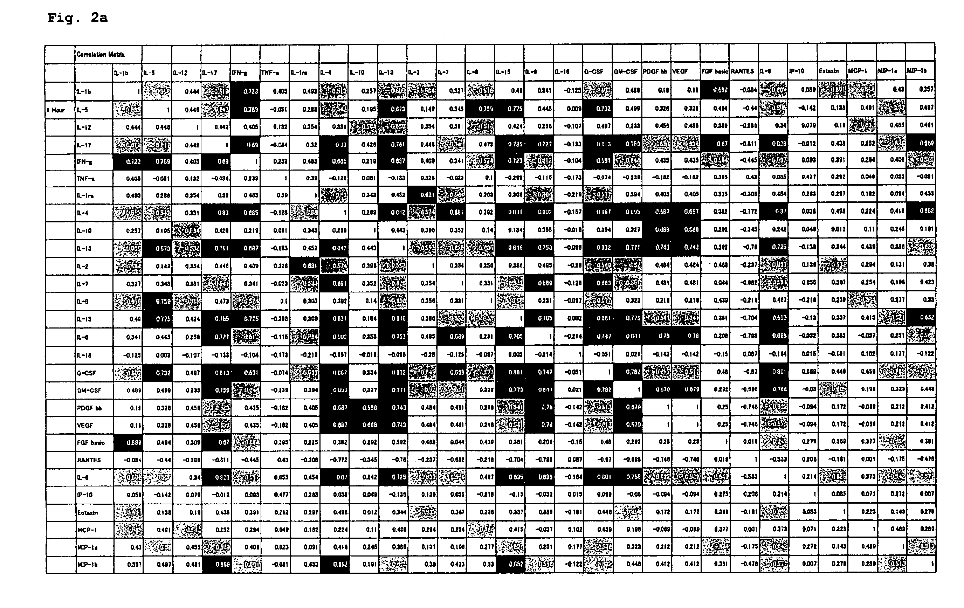

[Fig. 2a] Fig. 2a is a graph showing a correlation pattern of levels of blood plasma cytokines or chemokines after a 1 hour of Kraepelin load (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation, and the same applies to the other correlation tables described below).

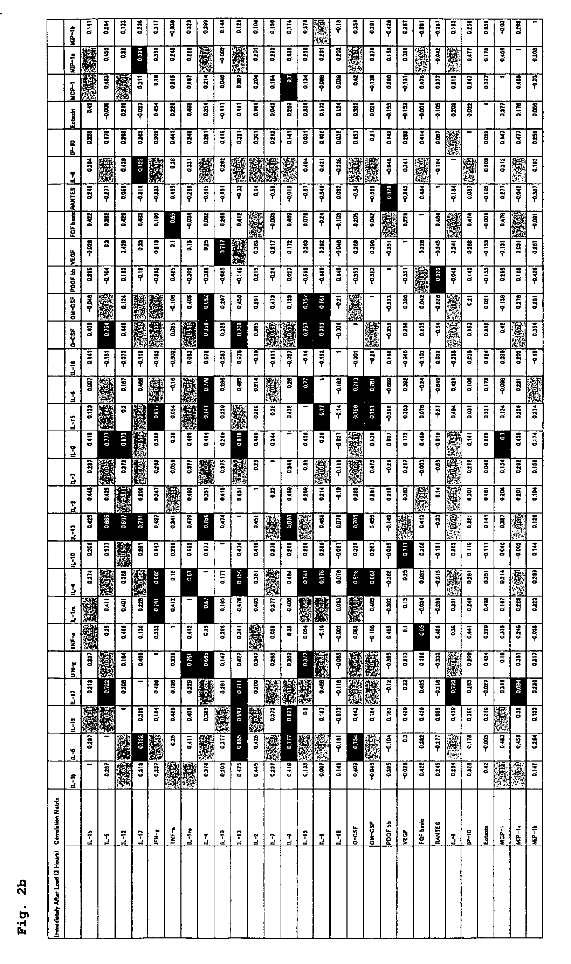

[Fig. 2b] Fig. 2b is a graph showing a correlation pattern of levels of blood plasma cytokines or chemokines after a 3 hours of Kraepelin load.

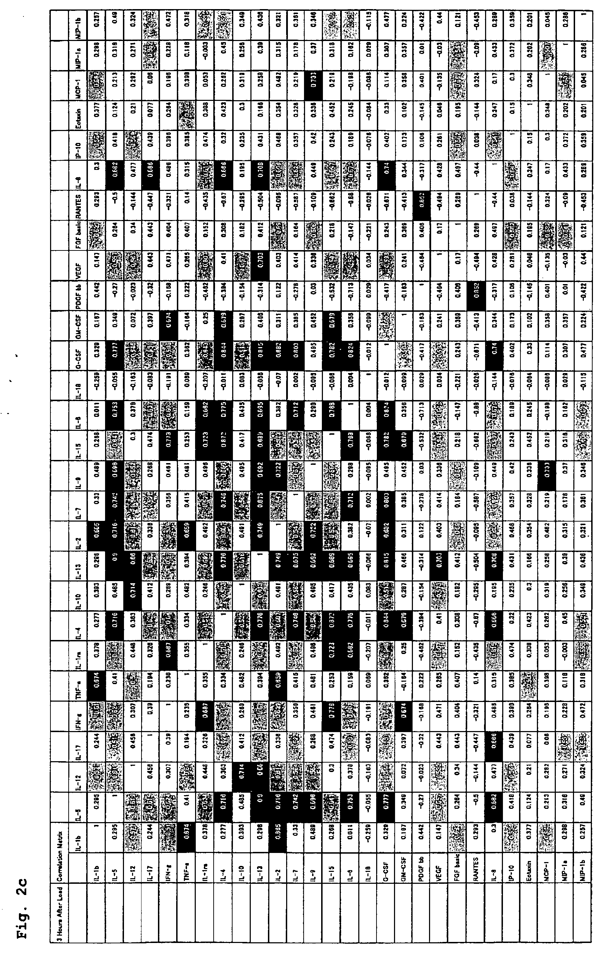

[Fig. 2c] Fig. 2c is a graph showing a correlation pattern of levels of blood plasma cytokines or chemokines after a 3 hours of Kraepelin load and a 3 hour rest after the load.

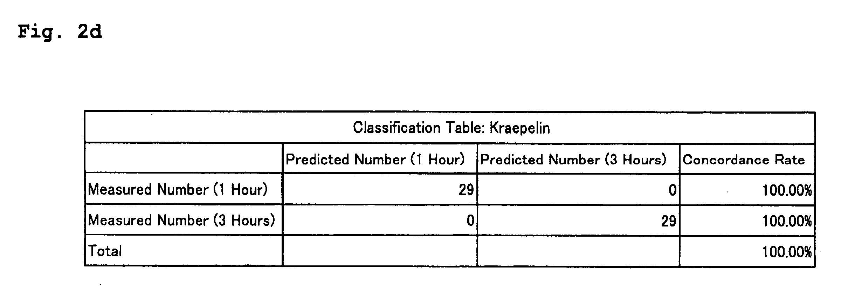

[Fig. 2d] Fig. 2d is a classification table obtained by a logistic regression analysis of the data on the 1 hour of Kraepelin load and the 3 hours of Kraepelin load.

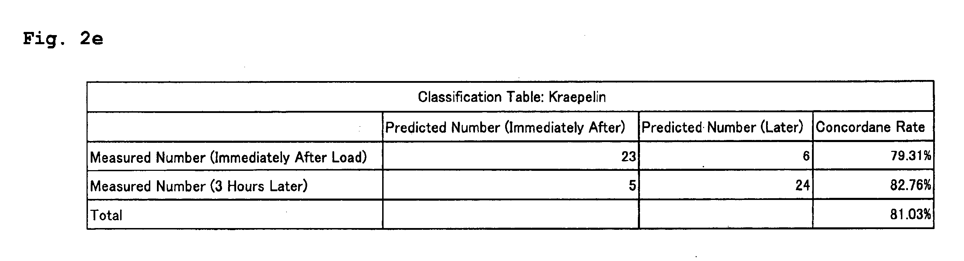

[Fig. 2e] Fig. 2e is a classification table obtained by the same analysis of the data on the 3 hours of Kraepelin load and the data after the 3 hour rest after the load.

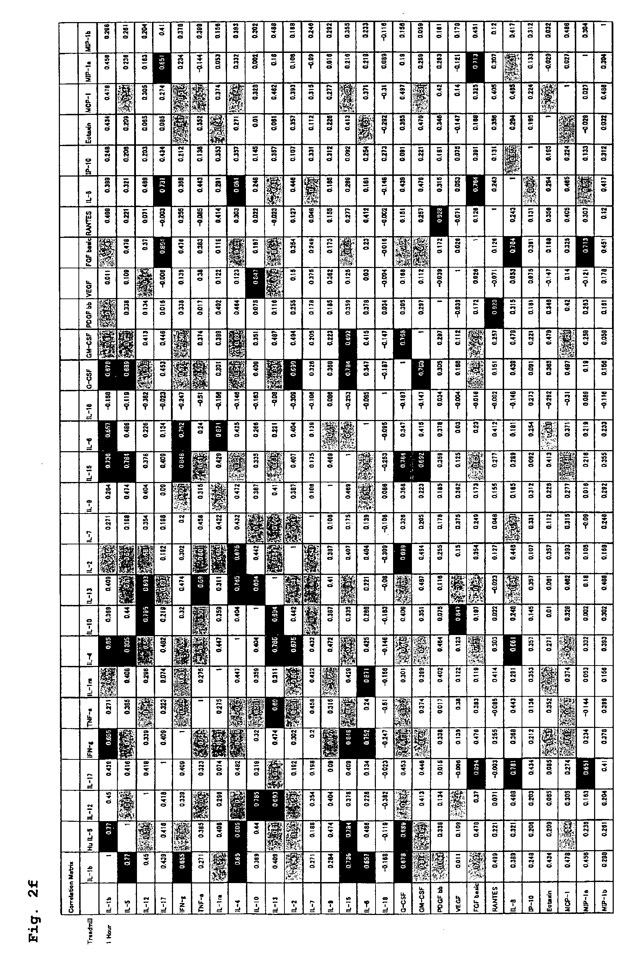

[Fig. 2f] Fig. 2f is a graph showing a correlation pattern of levels of blood plasma cytokines or chemokines after a 1 hour of treadmill exercise.

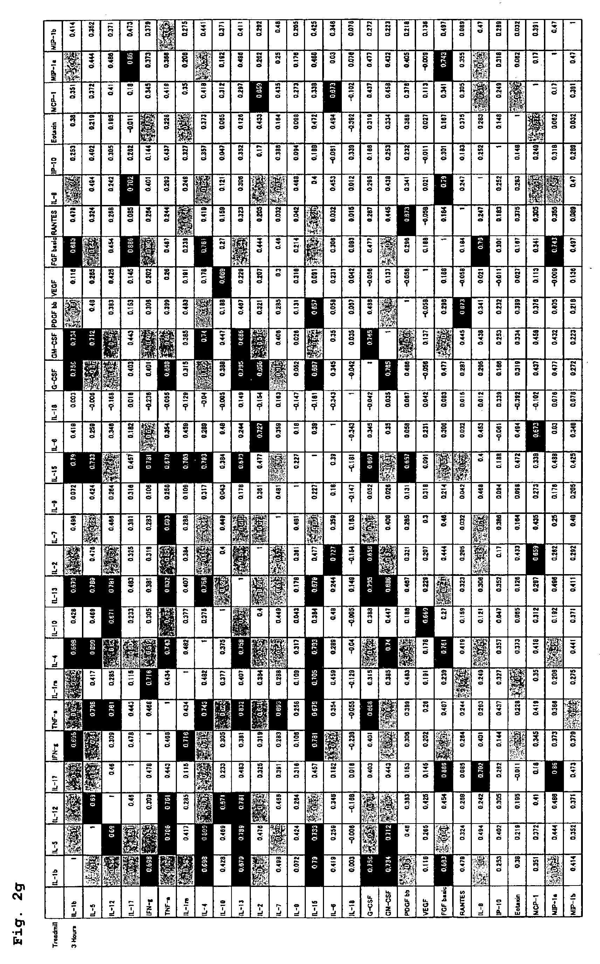

[Fig. 2g] Fig. 2g is a graph showing a correlation pattern of levels of blood plasma cytokines or chemokines after a 3 hours of treadmill exercise.

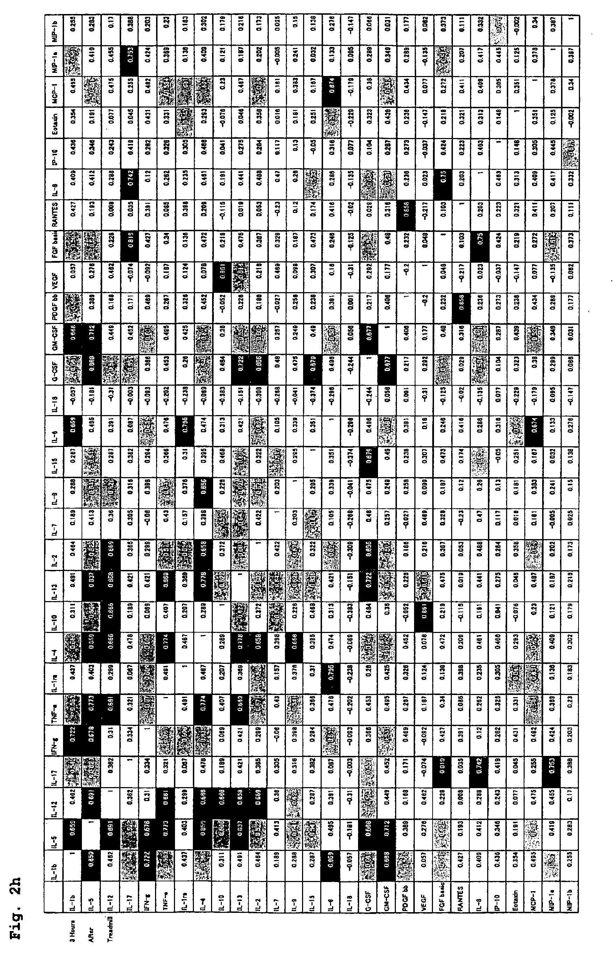

[Fig. 2h] Fig. 2h is a graph showing a correlation pattern of levels of blood plasma cytokines or chemokines after a 3 hours of treadmill exercise and a 3 hour rest after the exercise.

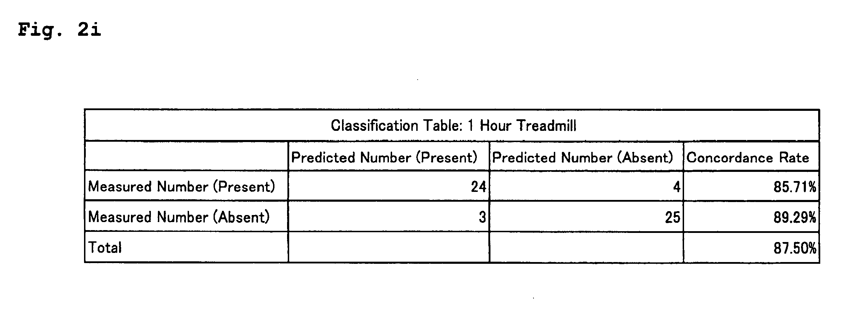

[Fig. 2i] Fig. 2i is a classification table obtained by a logistic regression analysis of the data before the treadmill exercise and the data after the 1 hour of treadmill exercise.

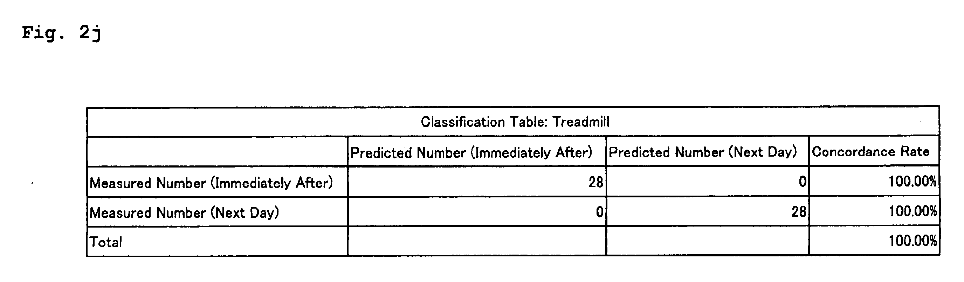

[Fig. 2j] Fig. 2j is a classification table obtained by the same analysis of the data after the treadmill exercise and the data on the next day.

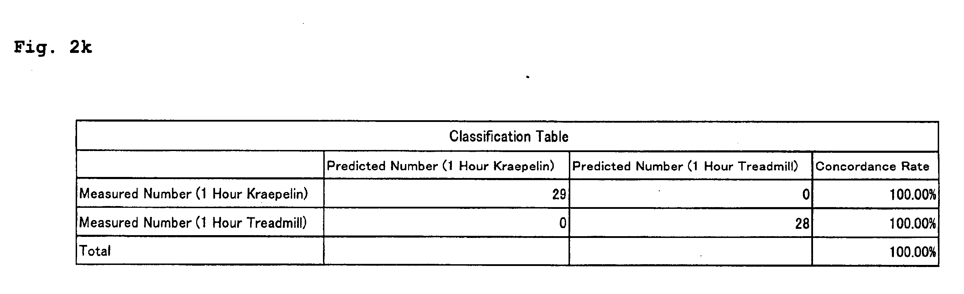

[Fig. 2k] Fig. 2k is a classification table obtained by the same analysis of the data on the 1 hour of Kraepelin load and the data on the 1 hour of treadmill exercise.

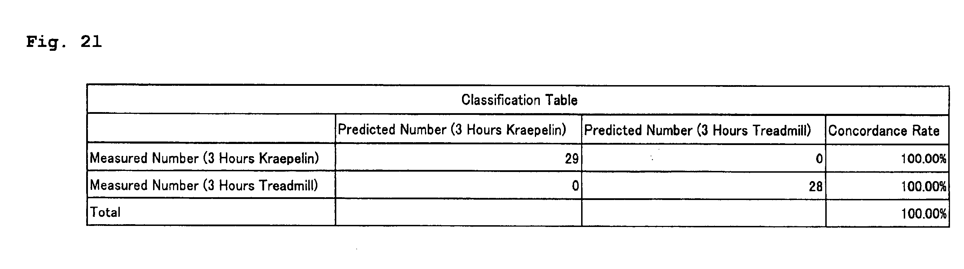

[Fig. 2l] Fig. 2l is a classification table obtained by the same analysis of the data on the 3 hours of Kraepelin load and the data on the 3 hours of treadmill exercise.

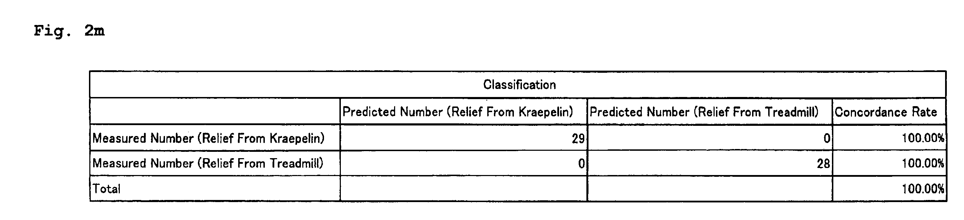

[Fig. 2m] Fig. 2m is a classification table obtained by the same analysis of the data of 3 hours of Kraepelin load followed by 3 hours of rest and the data of 3 hours of treadmill exercise followed by 3 hours of rest.

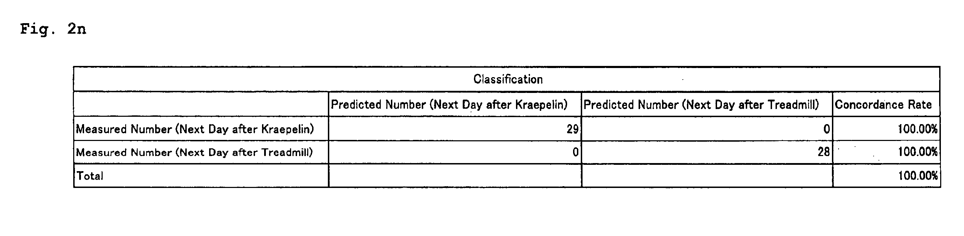

[Fig. 2n] Fig. 2n is a classification table between the group on the next day after the Kraepelin load and the group on the next day after the treadmill exercise.
[Fig. 3a] Fig. 3a is a graph showing a correlation pattern of levels of blood plasma cytokines or chemokines in patients with depression (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation).
[Fig. 3b] Fig. 3b is a classification table obtained by a logistic regression analysis to determine whether it is possible to correctly classify the subjects into a healthy group and a depression patient group according to the present invention.
[Fig. 4a] Fig. 4a is a graph showing a correlation pattern of levels of blood plasma cytokines or chemokines in patients with schizophrenia (with correlation coefficients of 0.50 to 0.6499 in light gray, 0.65 to 0.7999 in dark gray, 0.8 to 0.9999 in black; concerning the relative risk of correlation, p < 0.001 when the correlation coefficient is 0.50; in the table, darker shading indicates higher correlation).
[Fig. 4b] Fig. 4b is a classification table obtained by a logistic regression analysis to determine whether it is possible to correctly classify the subjects into a healthy group and a schizophrenia patient group according to the present invention.
[Fig. 4c] Fig. 4c is a classification table obtained by the analysis to determine whether it is possible to correctly classify the subjects into a depression patient group and a schizophrenia patient group according to the present invention.

### Best Mode for Carrying Out the Invention

In the present invention, the characteristics to be evaluated may be classified into the state of loads in a living organism and the pathological condition of a living organism. In the present invention, the evaluation or determination level is, in the case of a state, the state itself and the degree of manifestation of the state, and in the case of a pathological condition, the type and degree of the manifested pathological condition. Concerning pathological conditions which manifest themselves but for which no objective evaluation criterion has been conventionally found, the present invention also aims to provide criteria for evaluating such pathological conditions through the approach of monitoring potential conditions, specifically, variations in cytokines or chemokines in a living organism.

The indicating agent of stress or fatigue used in vitro in the present invention contains at least eight kinds of cytokines selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF.

As used herein, the term "indicating agent" refers to in vivo markers that serve to objectively indicate various mental conditions, for which only self-assessment evaluation approaches have conventionally been established. The "indicating agent" may also be in vivo markers that serve to objectively indicate the intensity of mental disorders.

As used herein, the term "biological load" means a chemical, physical, mental, verbal, or laborious load on the mental or physical condition of a living organism. The "biological load" is intended to include any endogenous constant load caused by pathological conditions in a living organism. In the present invention, for example, the biological load also includes the breakdown of biological homeostasis, loading of the breakdown of biological homeostasis, the precursor state of the breakdown of biological homeostasis, the breakdown of host-defense mechanism, loading of the breakdown of host-defense mechanism, a precursor state of the breakdown of host-defense mechanism.

As used herein, the term "stress" means various biological responses caused by the application of a chemical, physical, mental, verbal, or laborious temporary load to the mental or physical condition of a living organism. The "stress" also means various biological responses caused by the application of any endogenous constant load in a living organism.

As used herein, the term "fatigue" means any type of fatigue including physical fatigue and mental fatigue.

The cytokines contained in the indicating agent of the present invention are at least eight kinds selected from the above-mentioned group. To provide a more specific index of fatigue, they are 8 to 41 kinds, preferably 8 to 28 kinds, more preferably 8 to 20 kinds, and still more preferably 8 to 12 kinds.

In the present invention, "IL-1β" is a material of which the human amino acid sequence and base sequence are published under the Genbank Accession No. NM_000576 and which may be isolated or produced by publicly known methods. The "IL-1β" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-1 ra" is a material of which the human amino acid sequence and base sequence are published under the Genbank Accession No. NM_173841, NM_173842, NM_173843, NM_000577 and which may be isolated or produced by publicly known methods. The "IL-1 ra" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-2" is a material of which the human amino acid sequence and base sequence are published under the Genbank Accession No. NM_000586 and which may be isolated or produced by publicly known methods. The "IL-2" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-4" is a material of which the human amino acid sequence and base sequence are published under the Genbank Accession No. NM_000589, NM_172348 and which may be isolated or produced by publicly known methods. The "IL-4" also includes any congeners (such as homologues and splice variants), any variants, any derivatives, any mature forms, any analogues with amino acid modifications . Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-5" is a material of which the human amino acid sequence and base sequence are published under the Genbank Accession No. NM_000879 and which may be isolated or produced by publicly known methods. The "IL-5" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-6" is a material of which the human amino acid sequence and base sequence are published under the Genbank Accession No. NM_000600 and which may be isolated or produced by publicly known methods. The "IL-6" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-7" is a material of which the human amino acid sequence and base sequence are published under the Genbank Accession No. NM_000880 and which may be isolated or produced by publicly known methods. The "IL-7" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-8" is a material of which the human amino acid sequence and base sequence are published under the Genbank Accession No. NM_000584 and which may be isolated or produced by publicly known methods. The "IL-8" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-9" is a material of which the human amino acid sequence and base sequence are published under the Genbank Accession No. NM_000590 and which may be isolated or produced by publicly known methods. The "IL-9" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-10" is a material of which the human amino acid sequence and base sequence are published under the Genbank Accession No. NM_000572 and which may be isolated or produced by publicly known methods. The "IL-10" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-12" is a material of which the human amino acid sequence and base sequence are published under the Genbank Accession No. NM_002187, NM_000882 and which may be isolated or produced by publicly known methods. The "IL-12" also includes any congeners (such as homologues and splice variants), any variants, any derivatives any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-13" is a material of which the human amino acid sequence and base sequence are published under the Genbank Accession No. NM_002188 and which may be isolated or produced by publicly known methods. The "IL-13" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-15" is a material of which the human amino acid sequence and base sequence are published under the Genbank Accession No. NM_000585, NM_172174, NM_172175 and which may be isolated or produced by publicly known methods. The "IL-15" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-17" is a material of which the human amino acid sequence and base sequence are published under the Genbank Accession No. NM_002190 and which may be isolated or produced by publicly known methods. The "IL-17" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

"IL-18" is a material of which the human amino acid sequence and base sequence are published under the Genbank Accession No. NM_001562 and which may be isolated or produced by publicly known methods. The "IL-18" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "Eotaxin" is a material of which the human amino acid sequence and base sequence are published under the Genbank Accession No. D49372 and which may be isolated or produced by publicly known methods. The "Eotaxin" also includes any congeners (such as homologues and splice variants), any variants, any derivatives, any mature forms, any analogues with amino acid modifications . Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "FGF basic" is a material of which the human amino acid sequence and base sequence are published under the Genbank Accession No. NM_002006 and which may be isolated or produced by publicly known methods. The "FGF basic" also includes any congeners (such as homologues and splice variants), any variants, any derivatives, any mature forms, any analogues with amino acid modifications . Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids. The "FGF basic" is also referred to as "FGF-2".

In the present invention, "G-CSF" is a material of which the human amino acid sequence and base sequence are published under the Genbank and which may be isolated or produced by publicly known methods. The "G-CSF" also includes any congeners (such as homologues and splice variants), any variants, any derivatives, any mature forms, any analogues with amino acid modifications and the like. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "GM-CSF" is a material of which the human amino acid sequence and base sequence are published under the Genbank Accession No. X03021, M10633 and which may be isolated or produced by publicly known methods. The "GM-CSF" also includes any congeners (such as homologues and splice variants), any variants, any derivatives, any mature forms, any analogues with amino acid modifications.

Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IFN-γ" is a material of which the human amino acid sequence and base sequence are published under the Genbank Accession No. NM_000619 and which may be isolated or produced by publicly known methods. The "IFN-γ" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IP-10" is a material of which the human amino acid sequence and base sequence are published by the Genbank and which may be isolated or produced by publicly known methods. The "IP-10" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MCP-1" is a material of which the human amino acid sequence and base sequence are published by the Genbank and which may be isolated or produced by publicly known methods. The "MCP-1" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MIP-1α" is a material of which the human amino acid sequence and base sequence are published by the Genbank and which may be isolated or produced by publicly known methods. The "MIP-1α" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MIP-1β" is a material of which the human amino acid sequence and base sequence are published by the Genbank and which may be isolated or produced by publicly known methods. The "MIP-1β" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "PDGF-BB" is a material of which the human amino acid sequence and base sequence are published by the Genbank and which may be isolated or produced by publicly known methods. The "PDGF-BB" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "RANTES" is a material of which the human amino acid sequence and base sequence are published by the Genbank and which may be isolated or produced by publicly known methods. The "RANTES" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "TNF-α" is a material of which the human amino acid sequence and base sequence are published by the Genbank and which may be isolated or produced by publicly known methods. The "TNF-α" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "VEGF" is a material of which the human amino acid sequence and base sequence are published by the Genbank and which may be isolated or produced by publicly known methods. The "VEGF" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-3" is a material of which the human amino acid sequence and base sequence are published by the NCBI Accession No.: Hs.694 and which may be isolated or produced by publicly known methods. The "IL-3" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "IL-11" is a material of which the human amino acid sequence and base sequence are published by the Genbank Accession No.: NM_000641 and which may be isolated or produced by publicly known methods. The "IL-11" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the invention, "IFN-α" is a material of which the human amino acid sequence and base sequence are published by the Genbank and which may be isolated or produced by publicly known methods. The "IFN-α" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CSF-2" is a material of which the human amino acid sequence and base sequence are published by the Genbank and which may be isolated or produced by publicly known methods. The "CSF-2" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "TGF-β" is a material of which the human amino acid sequence and base sequence are published by the NCBI Accession No.: Hs.645227 and which may be isolated or produced by publicly known methods. The "TGF-β" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "neurotrophin 5" is a material of which the human amino acid sequence and base sequence are published by the NCBI Accession No.: Hs.534255 and which may be isolated or produced by publicly known methods. The "neurotrophin 5" also includes any congeners (such as homologues and splice variants), any variants, any derivatives, any mature forms, any analogues with amino acid modifications . Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "MCP-3" is a material of which the human amino acid sequence and base sequence are published by the Genbank Accession No.: X72309 and which may be isolated or produced by publicly known methods. The "MCP-3" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "β-2-microglobulin" is a material of which the human amino acid sequence and base sequence are published by the NCBI Accession No.: Hs.534255 and which may be isolated or produced by publicly known methods. The "β-2-microglobulin" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gbv/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "angiotensin II" is a material of which the human amino acid sequence and base sequence are publicly known and which may be isolated or produced by publicly known methods. The "angiotensin II" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CSF-3" is a material of which the human amino acid sequence and base sequence are published by the NCBI Accession No.: Hs.2333 and which may be isolated or produced by publicly known methods. The "CSF-3" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CXC chemokine ligand 1" is a material of which the human amino acid sequence and base sequence are published by Genbank and which may be isolated or produced by publicly known methods. The "CXC chemokine ligand 1" also includes any congeners (such as homologues and splice variants), any variants, any derivatives, any mature forms, any analogues with amino acid modifications . Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "CXC chemokine ligand 5" is a material of which the human amino acid sequence and base sequence are published by Genbank and which may be isolated or produced by publicly known methods. The "CXC chemokine ligand 5" also includes any congeners (such as homologues and splice variants), any variants, any derivatives, any mature forms, any analogues with amino acid modifications . Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, "HGF" is a material of which the human amino acid sequence and base sequence are published by the NCBI Accession No.: Hs.396530 and which may be isolated or produced by publicly known methods. The "HGF" also includes any congeners (such as homologues and splice variants), any variants, any derivatives, any mature forms, any analogues with amino acid modifications. Examples of its homologues include proteins of other species such as mice and rats corresponding to the human protein. Such homologues may be deductively identified with the base sequence of the gene identified according to HomoloGene (http://www.ncbi.nlm.nih.gov/HomoloGene/). Its variants include natural allelic variants, non-natural variants, and variants with amino acid sequence modifications produced by artificial deletion, substitution, addition, or insertion. The variants may have a homology of at least 70%, preferably of 80%, more preferably of 95%, even more preferably of 97%, with the original (non-variant) protein or (poly)peptide. Amino acid modifications include natural amino-acid modifications and non-natural amino-acid modifications and specifically include phosphorylations of amino acids.

In the present invention, cytokines constituting the indicating agent are preferably placed in different containers. In the present invention, the indicating agent may contain any other component, as long as it is mainly composed of the cytokines. Examples of other components include, but are not limited to, a solvent such as a buffer and a saline, a stabilizing agent, a bacteriostatic agent, and a preservative.

In the present invention, the use in vitro of an indicating agent serves to indicate that the physiological state of an animal is accompanied by stress or fatigue, preferably fatigue caused by mental stress. In particular, the degree of fatigue can be readily and quantitatively determined using the above-described cytokines, for the indicator, in a biological sample from an animal, preferably in blood plasma, serum, saliva, or urine. Specifically, at least eight cytokines constituting the indicating agent are preferably provided in amounts for indicating a normal level to a weak fatigue level or a strong fatigue level such that a comparison with samples can be made.

The agent for testing stress or fatigue characteristically contains at least eight kinds of antibodies selected from the group consisting of antibodies specifically recognizing IL-1β, IL-1ra, IL-2, IL-3, IL-4, IL-5, IL-6, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.

The antibodies may include polyclonal antibodies, monoclonal antibodies, chimeric antibodies, single-stranded antibodies, and antigen-binding part of the above antibodies, such as F(ab')₂ or Fab' fragments or fragments produced with Fab expression libraries.

The antibodies may be produced by conventional methods (Current Protocol in Molecular Biology, Chapter 11.12 - 11.13(2000)). Specifically, polyclonal antibodies may be produced by a process including the steps of immunizing non-human animals such as rabbits with any of the cytokines, which is expressed with E. coli or the like and purified by conventional techniques, or with a synthetic oligopeptide, having part of the amino acid sequence of any of the cytokines, by conventional techniques, and obtaining polyclonal antibodies from the serum of the immunized animals by conventional techniques. Alternatively, monoclonal antibodies may be produced by a process including the steps of immunizing non-human animals such as mice with any of the cytokines, which is expressed with E. coli or the like and purified by conventional techniques, or with an oligopeptide having part of the amino acid sequence of any of the cytokines, preparing hybridoma cells by cell fusion between the resulting spleen cells and myeloma cells, and culturing the hybridoma cells (Current protocols in Molecular Biology edit. Ausubel et al. (1987) Publish. John Wiley and Sons. Section 11.4 - 11.11). Chimeric antibodies may also be produced based on, for example, the techniques described in Jikken Igaku (Experimental Medicine), Extra Edition, Vol. 6, No. 10, 1988 or Japanese Patent Publication (JP-B) No. 03-73280. F(ab')₂ or Fab' may be produced by treating immunoglobulins with a proteolytic enzyme such as pepsin or papain.

The test agent may contain the antibody in a free form, a labeled form or an immobilized form. The test agent may also contain a carrier, which is generally contained in diagnostic agents. Examples of such a carrier include, but are not limited to, a preservative, a stabilizing agent, a buffer, a solvent such as water or a saline.

The present invention provides a method for measuring stress or fatigue, including the step of measuring the amounts of the cytokines in a biological sample with the test agent.

In the stress or fatigue measurement method, the amounts of the cytokines in a biological sample, preferably in blood plasma, serum, saliva, or urine, may be quantitatively measured with the antibodies in the test agent by publicly known methods. Systems that allow simple and simultaneous measurement of a number of proteins preferably include liquid-phase protein array systems (such as Bio-Plex (trade name) Suspension Array System (manufactured by Bio-Rad Laboratories)) in which protein recognition sensor-carrying microbeads are used to perform a binding reaction in a liquid suspension of the microbeads. When such array systems are used, measurement is possible in a wide range of a few pg/ml to several tens ng/ml.

The measured amount of each cytokine in the biological sample may be compared with the level of the stress or fatigue indicating agent so that the degree of stress or fatigue in the animal can be objectively and qualitatively or quantitatively evaluated.

The animal is preferably a vertebrate including human and particularly preferably a domestic or companion animal such as cattle, horse, pig, sheep, goat, chicken, dog, cat. The stress or fatigue measurement method of the invention may be applied to a domestic or companion animal. In the field of livestock or pet business where artificial rearing tends to cause stress, therefore, the state of stress or fatigue in an animal can be objectively monitored, which is advantageous for grasping and well controlling the health of the animal.

The antibodies may be placed in different containers. The antibodies placed in different containers may form a test kit for determining the intensity of mental conditions or disorders as described later. The test kit may include any other reagent such as a buffer for dilution of the reagent or the biological sample, a fluorescent dye, a reaction vessel, a positive control, a negative control, and test protocol instructions. The intensity of mental conditions or disorders can be easily measured using the kit.

The present invention can provide use in vitro of at least eight kinds of weighted cytokines selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, FGF-β, neurotrophin 5, MCP-3, β-2-microglobulin, angiotensin II, CSF-3, CXC chemokine ligand 1, CXC chemokine ligand 5 and HGF, as an indicating agent for evaluating mental conditions selected from the group consisting of mental fatigue, physical fatigue, stress, anxiety, depression and schizophrenia.

The at least eight kinds of weighted cytokines selected from the group consisting of IL-1β, IL-1ra, IL-2, IL-3, IL-4, IL-5, IL-6, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, FGF-β, neurotrophin 5, MCP-3, β-2-microglobulin, angiotensin II, CSF-3, CXC chemokine ligand 1, CXC chemokine ligand 5 and HGF, may be used in vitro as an indicating agent for evaluating intensity of mental disorders selected from the group consisting of mental fatigue, stress, depression, schizophrenia and anxiety.

All the indicating agents make it possible to evaluate not only conditions, disorders or development of diseases but also premorbid conditions (where physical examinations cannot reveal explicit diseases, but some pathological condition or any pathological sign exists so that the risk of development can be significantly predicted).

In the present invention, "stress" means various biological responses caused by the application of a chemical, physical, mental, verbal, or laborious temporary load to the mental or physical condition of a living organism. An index of specific stress caused by a specific load may be provided depending on how to weight the cytokines. Specifically, each cytokine may be multiplied by a weighting coefficient obtained by distribution calculation so that an index of stress for a mental condition or disorder can be provided.

In the present invention, "fatigue" includes physical fatigue and mental fatigue. Any of a physical fatigue-weighted index and a mental fatigue-weighted index may be provided depending on how to weight the cytokines. Specifically, each cytokine may be multiplied by a weighting coefficient obtained by distribution calculation so that an index of a mental disorder based on mental fatigue or an index of a mental condition caused by physical fatigue and mental fatigue can be provided.

As used herein, the term "depression" refers to the contents defined in DSM-IV and a group of diseases called "major depression" in the field of psychiatry.

In the present invention, the "schizophrenia" is as defined in DSM-IV.

In the present invention, the "anxiety" is as defined in DSM-IV.

In the present invention, in vitro use of the indicating agent of the intensity of mental conditions or disorders includes at least eight kinds of cytokines that are selected from the group described above and are each weighted. In order to make the indicating agent more specific to the intensity of each mental condition or disorder, 8 to 41 kinds of cytokines, preferably 8 to 28 kinds, more preferably 8 to 20 kinds, even more preferably 8 to 12 kinds should be selected.

In the present invention, cytokines constituting the indicating agent of the intensity of mental conditions or disorders are preferably placed in different containers. In the present invention, the indicating agent of mental conditions may contain any other component, as long as it is mainly composed of the cytokines. Examples of other components include, but are not limited to, a solvent such as a buffer and a saline, a stabilizing agent, a bacteriostatic agent, a preservative.

In vitro use of the indicating agent of the intensity of a mental condition or disorder according to the present invention may serve as an objective indicator in the field of animal mental health. In particular, cytokines in an animal biological sample, preferably in blood plasma, serum, saliva, or urine, may be each weighted to constitute the indicating agent so that various mental conditions can be readily and quantitatively determined.

In the present invention, the indicating agent of fatigue or the indicating agent of the intensity of a mental condition or disorder may comprise a combination of numerical values of weighted cytokines, namely a database. When the indicating agent is a database, various mental conditions can be readily and quantitatively determined for each item from the values measured with the test agent.

The agent for testing intensity of mental conditions or mental disorders characteristically contains at least eight kinds of molecule selected from the group consisting of antibodies specifically recognizing the aforementioned cytokines (IL-1β, IL-1ra, IL-2, IL-3, IL-4, IL-5, IL-6, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively).

The antibodies and other components contained in the agent for testing mental conditions or disorders may be the same as those contained in the agent for testing stress or fatigue.

A method for measuring the intensity of a mental condition or disorder, may include the steps of: measuring the amounts of cytokines in a biological sample with the agent for testing the intensity of a mental condition or disorder; and proportionally weighting each of the amounts obtained in the measuring step and comparing the weighted amounts with the indicating agent of the intensity of the mental condition or disorder.

In the method for measuring the intensity of a mental condition or disorder, the concentrations of the cytokines in a biological sample, preferably in blood plasma, serum, saliva, or urine, may be measured with the antibodies in the agent for testing the intensity of the mental condition or disorder, by publicly known methods. For example, the system that allows simple and simultaneous measurement of a number of proteins is preferably Bio-Plex Suspension Array System (manufactured by Bio-Rad Laboratories).

The value obtained by multiplying the measured amount of each cytokine in the biological sample by a coefficient is compared with the indicating agent of the mental condition so that the mental condition of an animal can be objectively and quantitatively evaluated.

The animal is preferably a vertebrate including human and particularly preferably a domestic or companion animal such as cattle, horse, pig, sheep, goat, chicken, dog, cat. The method for measuring the intensity of a mental condition or disorder may be applied to a domestic or companion animal. In the field of livestock or pet business where artificial rearing tends to cause stress, therefore, the intensity of the mental condition or disorder of an animal can be objectively monitored, which is advantageous for grasping the mental health of the animal.

A kit for testing the intensity of a mental condition or disorder may include the antibodies, preferably at least eight kinds of antibodies, placed in different compartments. The test kit may include any other reagent such as a buffer for dilution of the reagent or the biological sample, a fluorescent dye, a reaction vessel, a positive control, a negative control, test protocol instructions. Mental conditions can be easily measured using the test kit.

The levels of the cytokines in various mental conditions or disorders can be analyzed with high throughput. Individual differences can also be easily found based on the findings obtained by the present invention. Therefore, the present invention may be applied to discover new genes based on individual differences in the response of cytokine level to stress or may be applied to screening for stress resistance. The present invention may also be used to determine the anti-stress effect of anti-stress drugs. The present invention can provide the following advantages:
1. A set uniquely developed for stress indication may be used for measurement;
2. All the cytokines can be measured using 50 µl of a blood plasma or serum sample;
3. The entire process can be completed in about 6 hours; and
4. All of the resulting data may be compiled into a database, which can be easily statistically processed.

When the advantages are exploited for measurement of stress and so on, stress or other mental conditions can be grasped in a significantly inexpensive and speedy manner, as compared with conventional methods in which data are collected through an interview by a physician or psychologist and with a questionnaire and then compiled over several days to be evaluated (so that the result can vary between the facilities).

### Examples

The invention is more specifically described below with some examples and so on which are not intended to limit the scope of the invention. In the examples described below, before volunteer subjects underwent different tests and blood collection, approval of the ethical committee of the facility and then an informed consent were obtained in advance.

### Example 1: Scales of Mental Condition and Fatigue Condition

Psychological tests by questionnaire methods (SDS, MAS, GHQ, STAI, and CMI), a subjective symptom survey (prepared by Institute of Occupational Health), and surveys with a social phobia scale, an anthropophobia scale and a uniquely developed life situation questionnaire were performed on 402 volunteer subjects, so that it was confirmed that they were healthy. After the health check, 1 ml of blood was collected from each subject on a holiday morning and at the same time point after a night shift, respectively, and placed in an anticoagulant-containing blood collection spitz. Plasma was then separated from the blood under cooling at 4°C. The blood plasma was frozen and stored or stored on ice, and then the cytokines shown separately were simultaneously measured with a microbead protein array system (Bio-Plex Suspension Array System manufactured by Bio-Rad Laboratories and modified by ourselves). The cytokines were also measured by a conventional ELISA method, and the concentration of each cytokine in the blood was determined.

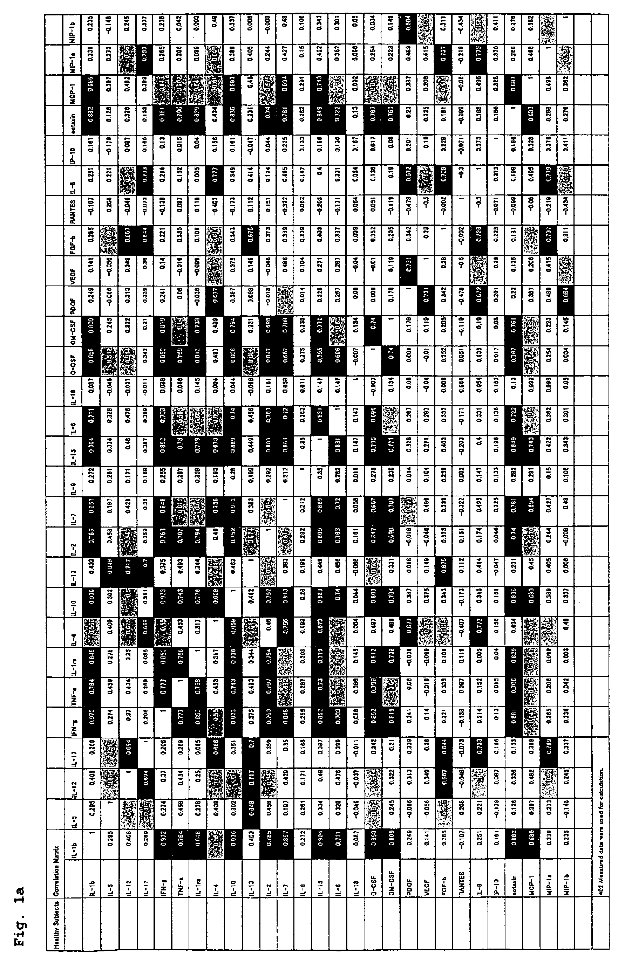

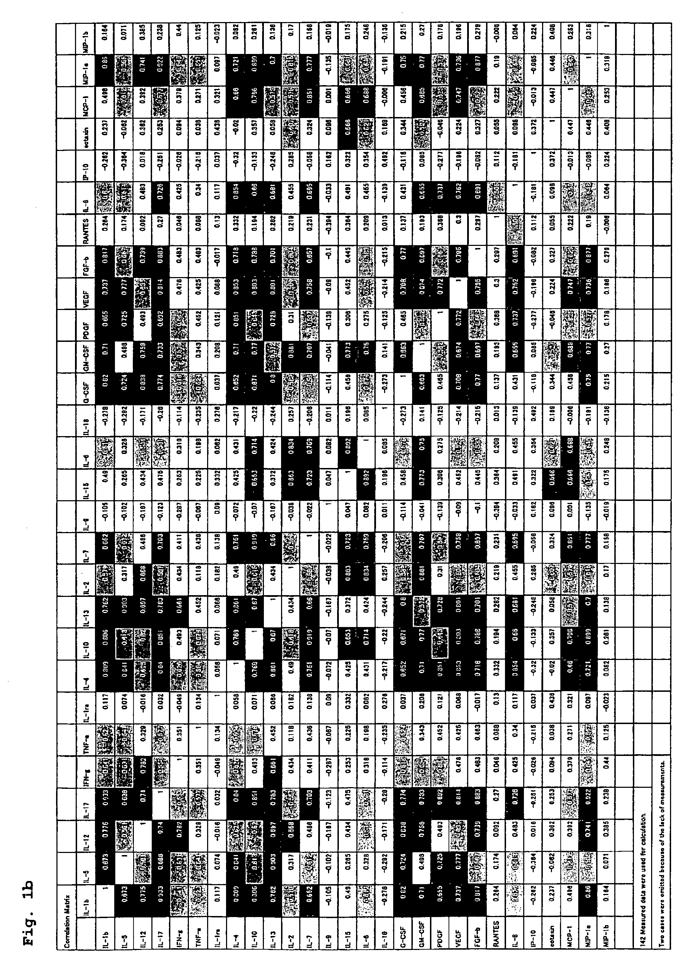

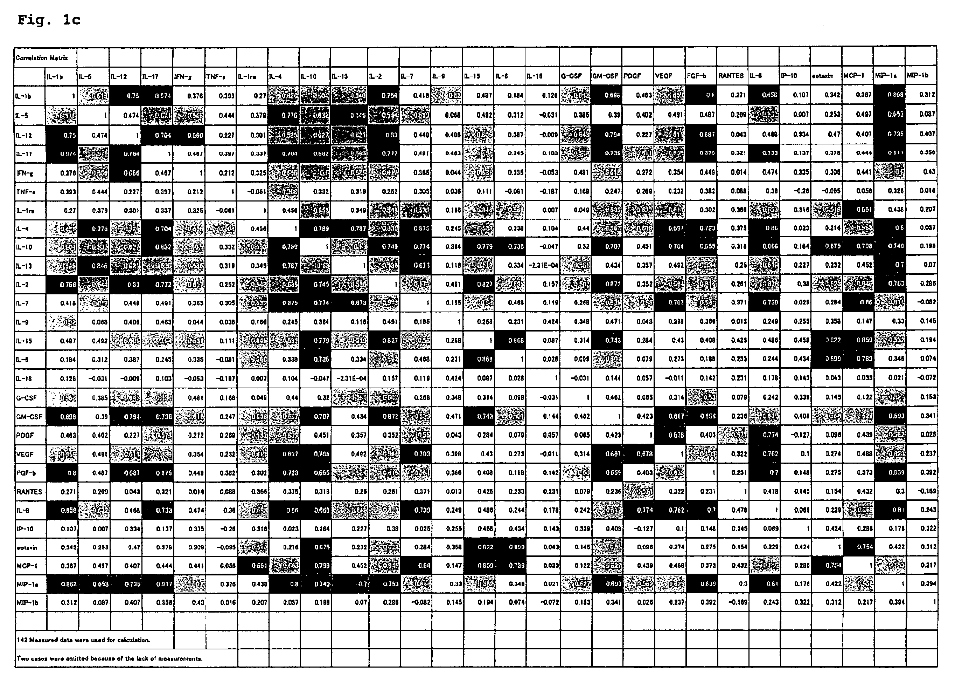

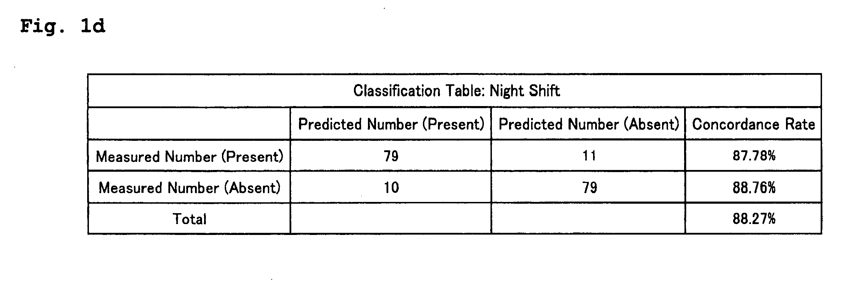

An example of the result is shown in Fig. 1a. The result was obtained by a process including determining the concentration of each cytokine in the blood, then calculating a correlation coefficient for each cytokine concentration in blood, and coloring high correlation values (with correlation coefficients of 0.50 to 0.6499 in light gray, 0.65 to 0.7999 in dark gray, 0.8 to 0.9999 in black; concerning the relative risk of correlation, p < 0.001 when the correlation coefficient was 0.50). The result shows that some cytokines have high correlation in the healthy subjects. Then, 142 subjects who were healthy but frequently work night shifts were examined as to whether the cytokine correlation could reflect the presence or absence of a night shift and was useful to discriminate between before and after a night shift. It is apparent that the correlation with the concentration of each cytokine in blood has changed between Fig. 1b (before a night shift) and Fig. 1c (after a night shift). It is recognized that the concentrations at the same time point after the night shift significantly deviated from normal values. Based on the cytokine correlation, a logistic regression analysis was performed on the data from 90 people randomly sampled from the subjects to determine whether discrimination between before and after a night shift was possible according to the present invention. The result is shown in Fig. 1d. In this example, the accuracy was 88%. Each cytokine value was multiplied by a certain coefficient for each of the items of depression, anxiety, fear, and stress in the psychological test, so that scores were produced. The resulting scores agreed with the results of interview by a psychiatrist more clearly than the psychological test, and therefore, it was possible to detect a tendency to depression, anxiety, or stress. This shows that different mental conditions (such as depression, excitement, tension, anxiety and the like) can be grasped and evaluated by the measurement method of the present invention.

### Example 2: Scale of Mental Fatigue or Physical Fatigue and Scale of Stress

Twenty-eight to 30 healthy volunteers underwent a Kraepelin test in which they continued simple calculation as a load on mental fatigue for 3 hours or a test in which an aerobic treadmill exercise to increase the 1-minute pulse rate to 180 was applied as a load on physical fatigue for 3 hours. Before and after each test, blood was collected and subjected to the measurement of cytokines in the same way as in Example 1. The cytokines were also measured in the same way 24 hours after the start of the load (21 hours after the end of the load). The result was obtained by calculating a correlation coefficient for each cytokine concentration in blood and coloring high correlation values.

The result showed that the correlation with the total cytokines changed before and after each test and during the recovery period. A comparison between the calculation for the mental load and the calculation for the treadmill exercise as the physical load showed that a certain combination of cytokines made a difference in the strength of the correlation and that fatigue and stress conditions can be grasped and evaluated by the measurement method of the present invention.

Based on the cytokine correlation, a logistic regression analysis was performed on the data from all the subjects to determine whether it was possible to discriminate between the Kraepelin test as a mental load and the treadmill exercise as a physical load. The result is shown in the drawings. The result showed that: it was possible to distinguish between a long loading time and a short loading time with respect to both the Kraepelin test and the treadmill test (in the example, the accuracy was 100% for the Kraepelin test and 81% for the treadmill test); it was also possible to show a difference in the recovery time between the subjects (in the example, the accuracy was 81% for the Kraepelin test and 100% for the treadmill test); and it was also possible to determine which load (Kraepelin load or treadmill load) was applied (in the example, the accuracy was 100%). This suggests that according to the present invention, not only the presence or absence of fatigue but also the type and level of fatigue can be classified, identified, or evaluated. It is also suggested that the improvement effect or effect of factors such as drugs, food products and living habits on mental fatigue or physical fatigue can be clearly shown using the accuracies shown in the classification tables obtained according to the present invention and taking into account that the recovery degree under the standard conditions may depend on drugs, food products, living habits and so on.

### Example 3: Scales for Diagnosis and Evaluation of Mental Disorders

Psychological tests by questionnaire methods (SDS, MAS, GHQ, STAI, SCID, and CMI), a subjective symptom survey (prepared by Institute of Occupational Health), and surveys with a social phobia scale, an anthropophobia scale and a uniquely developed life situation questionnaire were performed, as much as possible, on 160 volunteers diagnosed as having mental disorders according DSM-IV international diagnostic criteria.

Concurrently with the surveys, 1 ml of blood was collected from each volunteer and placed in an anticoagulant-containing blood collection spitz. Plasma was then separated from the blood under cooling at 4°C. The blood plasma was frozen and stored or stored on ice, and then the cytokines shown separately were simultaneously measured with a microbead protein array system (Bio-Plex Suspension Array System manufactured by Bio-Rad Laboratories and modified by ourselves). The cytokines were also measured by a conventional ELISA method, and the concentration of each cytokine in the blood was determined. On the other hand, all the volunteers each had a psychiatrically structured interview so that their psychological tendency and conditions were grasped.

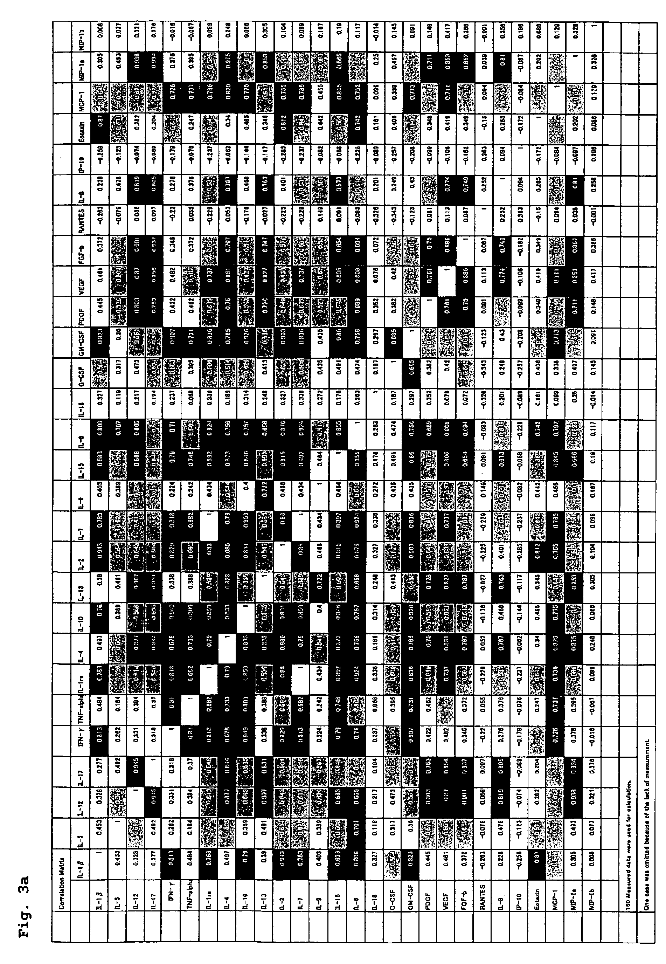

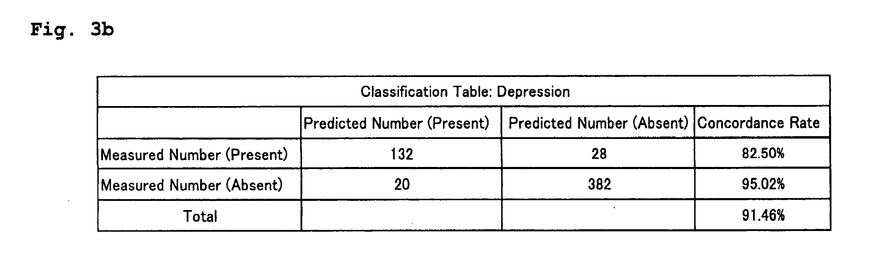

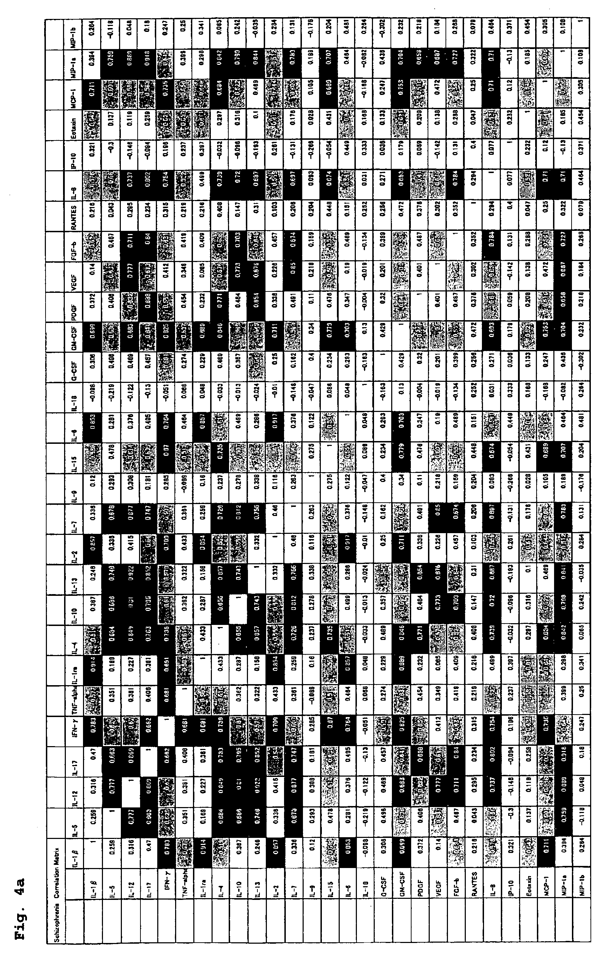

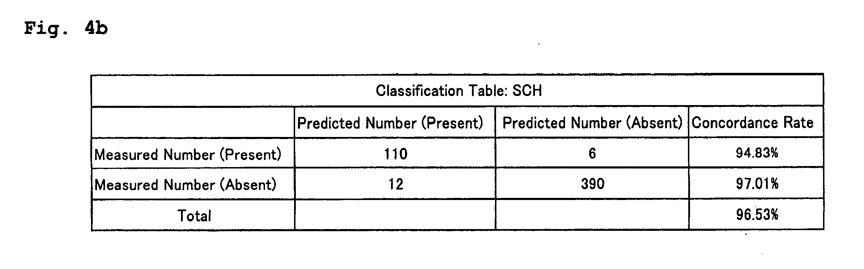

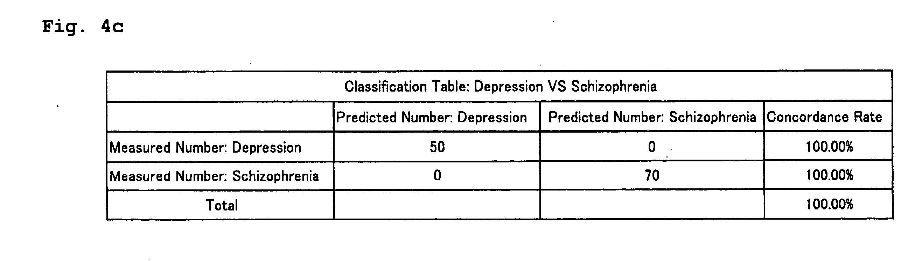

Each cytokine value was multiplied by a certain coefficient to produce scores. The resulting scores agreed with the results of interview by a psychiatrist more clearly than the psychological test, and therefore, it was possible to detect a tendency to mental conditions (depression, anxiety, stress, tension, and excitement). Although the psychological test scores are not disclosed for maintenance of the confidentiality of personal information of each patient, Fig. 3 shows cytokine correlation coefficients for patients with depression, and Fig. 4 shows cytokine correlation coefficients for patients with schizophrenia. The results were obtained by calculating a correlation coefficient for each cytokine concentration in blood and coloring high correlation values. It is apparent that the results each differ from the correlation coefficient table of the healthy subjects shown in Fig. 1. A logistic regression analysis was performed to determine whether it was possible to correctly diagnose depression according to the present invention. As a result, the accuracy was 91%.

A logistic regression analysis was also performed to determine whether it was possible to correctly diagnose schizophrenia according to the present invention. As a result, the accuracy was 96%.

The results show that different mental disorders (such as depression, schizophrenia, excitement, tension, anxiety and the like) can be grasped and evaluated by the cytokine measurement method according to the present invention. The results also show that psychiatric diagnosis or therapy validation, which would conventionally significantly depend on empirical determination and tend to be ambiguous, can be definitely made using the cytokine measurement and that the cytokine measurement would be useful for evaluation of therapeutic regimen efficacy. Mental conditions are often not externalized even though they are in a pathological state, and this adds to the problem in the current society. It has been found, however, that based on the above correlation found according to the present invention, diagnosis or assessment of depression and schizophrenia, two major diseases in the field of psychiatry, can be satisfactorily performed according to the present invention.

Predictions about dementia (senile dementia of Alzheimer type, Alzheimer disease and Pick disease) and central neurodegenerative diseases (OPCA, Parkinson disease, and diffuse Lewy body disease), if possible, should greatly contribute to the QOL of patients. Discrimination between depression, mild dementia and central neurodegenerative disease is possible at a stage where dementia or central neurodegenerative disease is not manifested. Therefore, it is expected that grouping of mild dementia or mild degenerative disease as proposed in the field of psychiatry can be achieved.

According to the present invention, the use of the correlation found between mental condition, fatigue or stress level and levels of the cytokines in blood, urine or saliva allows simple, inexpensive and objective evaluation of the risk or degree of stress, fatigue or dysthymia (particularly blues). The allocation of the score to each of the selected cytokines may be modified so that stress and fatigue can each be rated on an optimum scale in a single measurement procedure, and this is widely applicable.

Therefore, stress and fatigue can be objectively evaluated and their degree can be grasped. Since cytokines are causative agents or exacerbation factors in various diseases, care and preventive measures can be provided from the standpoint of the integration of mind and body.

### Industrial Applicability

According to the invention, the intensity of external loads or the effect of the loads can be evaluated or predicted, when temporary biological reactions occur. Specifically, external load-induced fatigue and relief from the fatigue can be evaluated, so that the individual's tendency to become fatigued or the individual's resilience can be evaluated. In addition, the degree of the effect of a certain cytokine on the individual's tendency to become fatigued or the individual's resilience can also be evaluated. Therefore, the application of the present invention to the field of occupational health may lead to prevention of occupational accidents. In addition, the present invention can contribute to the development of stress-relieving pharmaceuticals, food products, daily necessities, designs, images, sounds, buildings, residential environments, and so on.

According to the present invention, in vitro evaluation of disease states, in vitro recognition of potential pathological conditions, and in vitro detection of diseases before the manifestation of subjective symptoms are possible in a living organism with constant pathological conditions. Therefore, the present invention allows early detection and early treatment of pathological conditions, evaluation of treatment effects, determination of whether therapies are effective or ineffective, and unified detection and management of many pathological conditions (such as skin disorders caused by ultraviolet rays and so on, atopic dermatitis, psoriasis, acne vulgaris, pemphigus, ichthyosis, optic hyperesthesia, burns including inflammatory skin changes as pathological conditions, radiation dermatosis, Alzheimer disease, senile dementia of Alzheimer type, diffuse Lewy body disease, Pick disease, Binswanger disease, Parkinson disease, Parkinson syndrome, cerebral ischemia, cerebral ischemia/reperfusion injury, carbon monoxide poisoning, thinner poisoning, hemorrhagic encephalopathy of the newborn, hypoxic encephalopathy, hypertensive encephalopathy, epilepsy, multiple sclerosis, HIV encephalopathy, cerebral circulation disorder, cerebral vascular accident, disturbance of circadian rhythm, disturbance of appetite, pituitarism such as Addison disease, acromegaly and hypogonadism, thyroid dysfunction, obesity, abnormal control of blood sugar level, primary aldosteronism, adrenal dysfunction such as Cushing disease, osteodystrophy, inflammatory diseases, autoimmune diseases, dysregulation of host-defense systems, diseases with joint inflammation as the core pathological condition, such as rheumatoid arthritis, gout and arthritis, renal failure, fluid dysregulation, disruption of the balance between potassium, sodium and chloride ions, edema, impaired glucose tolerance such as diabetes, emaciation, convulsion, heart failure, pulmonary edema, hypoproteinemia, bleeding tendency, defluxion of renal uriniferous tubule epithelium, diseases with inflammation as the core pathological condition, abnormality in menstruation, endometriosis, and pituitary dysfunction-induced diseases such as loss of sexual desire). The present invention can contribute to not only an improvement in the efficiency of treatment of pathological conditions but also an improvement in the efficiency of preventive medicine. According to the present invention, selection of test groups is facilitated in the development of drugs or food products.

## Claims

1. Use in vitro of an indicating agent in evaluating mental conditions selected from the group consisting of mental fatigue, physical fatigue, stress, anxiety, depression and schizophrenia, which indicating agent comprises at least eight kinds of cytokines selected from the group consisting of IL-1β, IL-1ra, IL-2, IL-3, IL-4, IL-5, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, wherein the amount of each cytokine is multiplied by a weighting coefficient.

2. Use in vitro of a test agent in testing mental conditions, which mental conditions are selected from the group consisting of mental fatigue, physical fatigue, stress, anxiety, depression and schizophrenia, which test agent comprises at least eight kinds of antibodies selected from the group consisting of antibodies specifically recognizing IL-1β, IL-1ra, IL-2, IL-3, IL-4, IL-5, IL-7, IL-8, IL-9, IL-10, IL-11, IL-12, IL-13, IL-15, IL-17, Eotaxin, FGF basic, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophin 5, MCP-3, β-2-microglobulin, angiotensin II, CSF-3, CXC chemokine ligand 1, CXC chemokine ligand 5 and HGF, respectively.

3. A method of measuring mental conditions selected from the group consisting of mental fatigue, physical fatigue, stress, anxiety, depression and schizophrenia, which comprises a step of measuring the level of the cytokines in a biological sample using the test agent of claim 2, and a step of comparing the amount obtained in the aforementioned measurement step with that of the indicating agent of claim 1 by proportional weighting.

4. The method of claim 3, wherein the aforementioned biological sample is plasma, serum, saliva or urine.

## Patentansprüche

1. Verwendung eines Indikators in vitro bei der Bewertung von Geisteszuständen, die aus der Gruppe ausgewählt sind, die aus geistiger Erschöpfung, körperlicher Erschöpfung, Stress, Angstzuständen, Depression und Schizophrenie besteht, wobei der Indikator wenigstens acht Arten von Cytokinen umfasst, die aus der Gruppe ausgewählt sind, die aus IL-1β, IL-1ra, IL-2, IL-3, IL-4, IL-5, IL-7, IL-8, IL-9, IL-10, IL-11, IL-12, IL-13, IL-15, IL-17, Eotaxin, basischem FGF, G-CSF, GM-CSF, IFN-γ, IFN-α, IP-10, MCP-1, MIP-1α, MIP-1β, PDGF-BB, RANTES, TNF-α, VEGF, CSF-2, TGF-β, Neurotrophin 5, MCP-3, β-2-Microglobulin, Angiotensin II, CSF-3, CXC-Chemokin-Ligand 1, CXC-Chemokin-Ligand 5 und HGF besteht, wobei die Menge des Cytokins jeweils mit einer Gewichtungskoeffizienten multipliziert wird.

2. Verwendung eines Testreagens in vitro beim Testen von Geisteszuständen, wobei die Geisteszustände aus der Gruppe ausgewählt sind, die aus geistiger Erschöpfung, körperlicher Erschöpfung, Stress, Angstzuständen, Depression und Schizophrenie besteht, wobei das Testreagens wenigstens acht Arten von Antikörpern umfasst, die aus der Gruppe ausgewählt sind, die aus Antikörpern besteht, die spezifisch IL-1β, IL-1ra, IL-2, IL-3, IL-4, IL-5, IL-7, IL-8, IL-9, IL-10, IL-11, IL-12, IL-13, IL-15, IL-17, Eotaxin, basischem FGF, G-CSF, GM-CSF, IFN-γ, IFN-α, IP-10, MCP-1, MIP-1α, MIP-1β, PDGF-BB, RANTES, TNF-α, VEGF, CSF-2, TGF-β, Neurotrophin 5, MCP-3, β-2-Microglobulin, Angiotensin II, CSF-3, CXC-Chemokin-Ligand 1, CXC-Chemokin-Ligand 5 bzw. HGF erkennen.

3. Verfahren zum Messen von Geisteszuständen, die aus der Gruppe ausgewählt sind, die aus geistiger Erschöpfung, körperlicher Erschöpfung, Stress, Angstzuständen, Depression und Schizophrenie besteht, umfassend einen Schritt des Messens der Konzentration von Cytokinen in einer biologischen Probe unter Verwendung des Testreagens von Anspruch 2 und einen Schritt des Vergleichens der in dem oben genannten Schritt des Messens erhaltenen Menge mit der Menge des Indikators von Anspruch 1 durch proportionales Gewichten.

4. Verfahren gemäß Anspruch 3, wobei es sich bei der oben genannten biologischen Probe um Plasma, Serum, Speichel oder Urin handelt.

## Revendications

1. Utilisation in vitro d'un agent indicateur pour évaluer des maladies mentales choisies dans le groupe constitué de la fatigue mentale, de la fatigue physique, du stress, de l'anxiété, de la dépression et de la schizophrénie, l'agent indicateur comprenant au moins huit types de cytokines choisies dans le groupe constitué des IL-1β, IL-1ra, IL-2, IL-3, IL-4, IL-5, IL-7, IL-8, IL-9, IL-10, IL-11, IL-12, IL-13, IL-15, IL-17, éotaxine, FGF basique, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophine 5, MCP-3, β-2-microglobuline, angiotensine II, CSF-3, chimiokine CXC Ligand 1, chimiokine CXC Ligand 5 et HGF, dans laquelle la quantité de chaque cytokine est multipliée par un coefficient de pondération.

2. Utilisation *in vitro* d'un agent d'analyse pour analyser des maladies mentales choisies dans le groupe constitué de la fatigue mentale, de la fatigue physique, du stress, de l'anxiété, de la dépression et de la schizophrénie, l'agent d'analyse comprenant au moins huit types d'anticorps choisis dans le groupe constitué d'anticorps reconnaissant spécifiquement les IL-1β, IL-1ra, IL-2, IL-3, IL-4, IL-5, IL-7, IL-8, IL-9, IL-10, IL-11, IL-12, IL-13, IL-15, IL-17, éotaxine, FGF basique, G-CSF, GM-CSF, IFN-γ, IFN-α, IP-10, MCP-1, MIP-1α, MIP-1β, PDGF-BB, RANTES, TNF-α, VEGF, CSF-2, TGF-β, neurotrophine 5, MCP-3, β-2-micro-globuline, angiotensine II, CSF-3, chimiokine CXC Ligand 1, chimiokine CXC Ligand 5 et HGF, respectivement.

3. Procédé pour mesurer les maladies mentales choisies dans le groupe constitué de la fatigue mentale, de la fatigue physique, du stress, de l'anxiété, de la dépression et de la schizophrénie, qui comprend une étape consistant à mesurer le taux de cytokines dans un échantillon biologique avec l'agent d'analyse de la revendication 2 et une étape consistant à comparer la quantité obtenue à l'étape de mesure susmentionnée avec celle de l'agent indicateur de la revendication 1 par pondération proportionnelle.

4. Procédé selon la revendication 3, dans lequel l'échantillon biologique est du plasma, du sérum, de la salive ou de l'urine.
